# EUROPÄISCHE PATENTSCHRIFT

(11) **EP 1 032 329 B1**
(45) Veröffentlichungstag und Bekanntmachung des Hinweises auf die Patenterteilung: **07.07.2004**
(21) Anmeldenummer: 98963470.4
(22) Anmeldetag: 17.11.1998
(51) Int. Cl.: A61F 2/06

(54) **STENT ZUR IMPLANTATION IM MENSCHLICHEN KÖRPER, INSBESONDERE IN BLUTGEFÄSSE**
STENT FOR IMPLANTATION IN THE HUMAN BODY, ESPECIALLY IN BLOOD VESSELS
EXTENSEUR POUR OPERATIONS D'IMPLANTATION DANS LE CORPS HUMAIN, NOTAMMENT DANS DES VAISSEAUX SANGUINS

(30) Priorität: 18.11.1997 DE 19750971
(43) Veröffentlichungstag der Anmeldung: 06.09.2000
(73) Patentinhaber: Schneider (Europe) GmbH, 8180 Bülach (CH)
(72) Erfinder: BURLAKOV, Oleg Afanasevich, Moskau (RU); SACHZERINSKAYA, Natalya Alexandrovna, Ljuberezker Kreis, Moskau (RU); PIERER, Wolfgang, D-09126 Chemnitz (DE)
(74) Vertreter: Heuckeroth, Volker, Dr.
(86) Internationale Anmeldenummer: PCT/EP1998/007359
(87) Internationale Veröffentlichungsnummer: WO 1999/025271

(56) Entgegenhaltungen:
- EP-A- 0 740 928
- EP-A- 0 744 164
- WO-A-97/09945

## Beschreibung

Die Erfindung betrifft einen Stent zur Implantation im menschlichen Körper, insbesondere in Blutgefäße, mit einem hohlzylindrischen Körper, der aus mehreren umfänglich gegeneinander versetzten fadenförmigen Elementen aufgebaut ist, die zu einem Geflecht mit einer Vielzahl polygonförmiger Zellen verflochten sind, wobei jedes fadenförmige Element von einem ersten Ende des Stents ausgehend entsprechend dem Geflecht zum anderen Ende des Stents geführt ist, dort in Richtung erstes Ende umgelenkt und wieder zum ersten Ende entsprechend dem Geflecht rückgeführt ist, wobei freie Endabschnitte der fadenförmigen Elemente an dem ersten Ende auflösungssicher miteinander verbunden sind, und wobei jedes fadenförmige Element an dem Ende, an dem es umgelenkt ist, zu einer Schlaufe gelegt ist.

Ein derartiger Stent ist aus der EP-A-0 744 164 bekannt.

Unter einem Stent wird allgemein eine selbstexpandierende Endoprothese zur Offenhaltung gangartiger Strukturen im menschlichen Körper, bspw. von Blutgefäßen wie Venen oder Arterien, verstanden. Durch Implantation eines Stents soll einer Verengung des entsprechenden Gefäßes, die z.B. durch Druck von außen bedingt sind, entgegengewirkt werden.

Die Stents zur Implantation in Blutgefäße weisen einen hohlzylindrischen Körper auf, dessen Außendurchmesser in etwa dem Innendurchmesser des jeweiligen Blutgefäßes, in das der Stent implantiert werden soll, entspricht. Der Körper des Stents ist somit in Längsrichtung für den Durchtritt von Blut offen. Der Körper ist aus mehreren gegeneinander versetzten fadenförmigen Elementen aufgebaut, die zu einem Geflecht mit einer Vielzahl polygonförmiger Zellen verflochten sind. Das Geflecht kann so aufgebaut sein, daß zwei sich kreuzende Systeme der fadenförmigen Elemente so miteinander verkreuzt sind, daß jedes fadenförmige Element des einen Systems abwechselnd über und unter jedem fadenförmigen Element des anderen Systems geführt ist. Eine solche Bindung des Geflechts wird als Leinwand-Bindung bezeichnet.

Der Stent läßt sich auf einem Applikator in seiner Längsrichtung strecken, wodurch sich der Durchmesser des Stents zur Implantation verringern läßt. Mittels des Applikators wird der Stent im gestreckten bzw. gespannten Zustand in das Gefäß implantiert. Nachdem der Stent durch den Applikator an der gewünschten Stelle in dem Gefäß positioniert ist, wird der Applikator entfernt. Da nunmehr keine äußeren Längsdehnungskräfte auf den Stent wirken, relaxiert der Stent aufgrund seiner zellenförmigen Struktur elastisch in seine ursprüngliche Länge, dehnt sich dabei radial und schmiegt sich an die Gefäßinnenwand an. Dies kann noch dadurch verstärkt werden, daß Materialien mit einem Formgedächtnis verwendet werden.

Herkömmliche Stents werden dadurch hergestellt, daß mehrere fadenförmige Elemente fortlaufend in der Art einer Meterware zu einem endlosen rohrförmigen Körper miteinander verflochten werden. Zur Fertigung der einzelnen Stents wird dann das rohrförmige Geflecht abgelängt, d.h. das Geflecht wird entsprechend der vorbestimmten Länge des herzustellenden Stents in einzelne Stücke geschnitten. Dabei entstehen Stents, an deren beiden Enden die freien Enden der fadenförmigen Elemente etwa axial abstehen und unfixiert sind. Da die einzelnen fadenförmigen Elemente an den Verkreuzungspunkten nicht aneinander fixiert sind, besteht bei einem solchen Stent der Nachteil darin, daß sich die fadenförmigen Elemente im Bereich der geschnittenen Enden des Stents voneinander lösen können, d.h. daß sich die fadenförmigen Elemente an den Enden wieder teilweise entflechten.

Es ist daher erforderlich, daß die überstehenden Enden nach dem Zuschneiden des Geflechts aneinander fixiert werden. Dies geschieht bei den herkömmlichen Stents bspw. dadurch, daß die einzelnen Stents anschließend mit einem Überzug, bspw. aus Latex, überzogen werden. Aber auch eine solche Fixierung der Enden des Stents ist nicht ausreichend, um eine Auflösung des Geflechts zu verhindern. Eine Auflösung des Geflechts im Bereich der Enden des Stents muß jedoch vermieden werden, damit der Stent auch im Bereich seiner Enden eine genügend hohe Steifigkeit aufweist, um das Gefäß offenzuhalten, und außerdem sicher an der Stelle in dem Gefäß, an der er implantiert ist, verankert ist und von dem in dem Gefäß strömenden Blut nicht verschoben wird. Bei einer Auflösung des Geflechts ist dies jedoch nicht mehr gewährleistet.

Eine Auflösung des Geflechts bei unzureichender Fixierung im Bereich der Enden des Stents kann insbesondere dann auftreten, wenn der Stent zur Implantation in das Gefäß auf dem Applikator in Längsrichtung gespannt wird. Die zum Zusammendrücken des Stents auf diesen radial einwirkenden Druckkräfte führen zu einer Längenschubdehnung des Stents. Dabei besteht die Gefahr, daß sich das Geflecht beim Spannen an seinen Enden aufzieht, d.h. daß der Stent an seinen Enden seine polygonförmige Zellenstruktur verliert. Beim Einführen des Stents in das Gefäß können die freien Enden der fadenförmigen Elemente außerdem ein Hindernis darstellen, insbesondere wenn der Stent aus Draht hergestellt ist, da sich die freien überstehenden Enden seitlich abbiegen und in die Gefäßinnenwand bohren können, so daß sich der Stent in dem Gefäß nicht mehr an die gewünschte Stelle verschieben läßt. Außerdem kann dabei die Gefäßinnenwand beim Implantieren des Stents durch die freiliegenden freien Enden der fadenförmigen Elemente verletzt werden.

Bei dem aus der bereits zuvor genannten EP-A-0 744 164 bekannten Stent wird dieses Problem dadurch beseitigt, daß jedes fadenförmige Element von einem ersten Ende des Stents ausgehend entsprechend dem Geflecht zum anderen Ende des Stents geführt ist, dort in Richtung erstes Ende umgelenkt und wieder zum ersten Ende entsprechend dem Geflecht rückgeführt ist, wobei freie Endabschnitte der fadenförmigen Elemente an dem ersten Ende miteinander verdrillt sind.

Bei diesem bekannten Stent sind die einzelnen fadenförmigen Elemente an dem Ende, an dem jedes fadenförmige Element in Richtung gegenüberliegendes Ende umgelenkt ist, jeweils zu einer Schlaufe gelegt, die durch eine Drehung um 180° um die Längsachse des Stents verdrillt ist. Auf diese Weise können die Schlaufen keinen Beitrag zur radialen Expansionskraft des Stents leisten, da die in die gebogenen Schlaufen eingebrachte Spannenergie sich wegen der Drahtverwindungen nicht auf die in Umfangsrichtung erstreckenden Drahtabschnitte übertragen kann.

Aus der WO-A-97/09945 ist ein weiterer Stent bekannt, bei dem das Geflecht der fadenförmigen Elemente eine Zellenstruktur aufweist, das herzförmige bzw. pfeilspitzenförmige einzelne Zellen aufweist, wobei jeweils zwei miteinander verbundene kürzere Zellseiten zwei wechselseitig konvergierenden längeren Zellseiten gegenüberliegen und mit diesen verbunden sind. An beiden Enden des Stents sind die fadenförmigen Elemente verdrillt.

Der Erfindung liegt daher die Aufgabe zugrunde, einen Stent der eingangs genannten Art dahingehend zu verbessern, daß die radiale Expansionskraft des Stents verbessert ist.

Hinsichtlich des eingangs genannten Stents wird diese Aufgabe dadurch gelöst, daß die Schlaufe so ausgebildet ist, daß die beiden von jeder Schlaufe wegführenden Enden des zugehörigen fadenförmigen Elementes im Bereich der Schlaufe eine Art Schenkelfeder bilden.

Bei dem erfindungsgemäßen Stent, bei dem die Schlaufen eine noch bessere Verankerung des erfindungsgemäßen Stents in dem Gefäß bewirken, weil die Umlenkungsstellen der fadenförmigen Elemente durch die Schlaufen stabiler ausgebildet sind, besteht ein besonderer Vorteil der Ausgestaltung der Schlaufe so, daß die beiden von jeder Schlaufe wegführenden Enden des zugehörigen fadenförmigen Elementes im Bereich der Schlaufe eine Art Schenkelfeder bilden, ähnlich wie bei einer Wäscheklammer, darin, daß die Schlaufen einen Beitrag zur radialen Expansionskraft des Stents leisten können. Die Schlaufen erzeugen dabei eine Rückstellkraft an dem einen Ende des Stents, die bestrebt ist, den Stent aufzuweiten. Dadurch wird die Expansionskraft insbesondere an dem einen Ende des Stents, an dem die fadenförmigen Elemente umgelenkt sind, vorteilhaft erhöht. Die Schlaufen können im einfachsten Fall dadurch gebildet sein, daß jedes fadenförmige Element im Bereich seiner Umlenkungsstelle leicht gespreizt U-förmig umgebogen ist.

Der erfindungsgemäße Stent kann Stent für Stent einzeln hergestellt werden und ist nicht aus einer rohrförmigen Meterware zugeschnitten. Dadurch, daß die fadenförmigen Elemente von dem ersten Ende des Stents ausgehend zum anderen Ende des Stents geführt sind, dort umgelenkt und wieder zum ersten Ende entsprechend dem Geflecht rückgeführt sind, weist der erfindungsgemäße Stent an den Umlenkungsstellen eine erste Fixierung des Geflechts auf, weil an den Umlenkungsstellen bereits jeweils zwei Enden der fadenförmigen Elemente einstückig miteinander verbunden sind. Zur Fixierung müssen die fadenförmigen Elemente an diesem Ende nicht durch Schweißen, Kleben oder dgl. verbunden werden. Die unauflösbar miteinander verbundenen freien Endabschnitte bilden an dem ersten Ende eine sichere Fixierung des Geflechtes, das somit auch an diesem Ende nicht aufgezogen werden kann. Eine unauflösbare Verbindung im Sinne der Erfindung kann durch Schweißen, Löten, Kleben, durch Befestigungsmittel wie Clips oder dgl. oder besonders vorteilhaft durch Verdrillen der freien Endabschnitte erreicht werden. Der erfindungsgemäße Stent läßt sich somit in das Blutgefäß implantieren, ohne daß sich dabei das Geflecht im Bereich der Enden des Stents aufzieht. Als fadenförmige Elemente können alle biegsamen, dünnen Materialien insbesondere in Form von Drähten verwendet werden.

In einer bevorzugten Ausgestaltung sind die fadenförmigen Elemente in Form einer Leinwand-Bindung zu dem Geflecht verflochten.

Bei dieser Art des Geflechtes sind die Fadenlagen derart miteinander verkreuzt, daß von den Umlenkungsstellen an dem einen Ende des Stents aus zum anderen Ende hin die beiden Enden jedes fadenförmigen Elements abwechselnd über und unter jedem Ende desselben und der anderen fadenförmigen Elemente geführt sind. Die polygonförmigen Zellen sind bei diesem Flechtmuster rhombenförmig ausgebildet. Diese Art eines Geflechtes eignet sich für Stents zur Implantation in Blutgefäße besonders. Bei einer solchen Art eines Geflechtes berühren sich die fadenförmigen Elemente nämlich jeweils nur punktförmig, wodurch die fadenförmigen Elemente an den Kreuzungsstellen frei beweglich bleiben und sich gegenseitig verschieben und verdrehen können. Die erfindungsgemäße Ausgestaltung des Geflechts ist somit axial und radial flexibel, und die Expansionskraft des Stents ist über den Flechtwinkel, die Windungsdichte, den Durchmesser und das Material der fadenförmigen Elemente einstellbar. Ein weiterer Vorteil des Geflechts nach Art einer Leinwandbindung besteht darin, daß es wesentlich schneller, einfacher und kostengünstiger herstellbar ist.

Dabei ist es bevorzugt, wenn die Schlaufe als Knoten ausgebildet ist.

Eine Ausbildung der Schlaufe als Knoten hat den Vorteil, daß der erfindungsgemäße Stent im Bereich der Umlenkungsstellen noch weiter verstärkt ist, wodurch sich eine noch bessere Verankerung des erfindungsgemäßen Stents in dem Blutgefäß ergibt.

Außerdem wird die Schlaufe durch den Knoten fixiert. Der Knoten ist vorteilhaft so ausgebildet, daß die zuvor genannte, eine Schenkelfeder bildende Ausgestaltung der Umlenkungsstellen erhalten bleibt.

In einer weiteren bevorzugten Ausgestaltung sind die freien Endabschnitte miteinander verdrillt.

Durch das Verdrillen sind die freien Endabschnitte zusätzlich vorteilhaft verstärkt, wodurch sie sich beim Einführen des erfindungsgemäßen Stents in das Blutgefäß querstellen können. Der erfindungsgemäße Stent läßt sich somit in das Gefäß schieben, ohne sich mit der Gefäßwand zu verhaken oder diese zu verletzen. Das Verdrillen der freien Endabschnitte stellt darüber hinaus eine leicht herstellbare unauflösbare Verbindung der freien Endabschnitte dar, weil keine weiteren Befestigungsmittel, wie Clips oder Klammern, oder Schweiß oder Lötmaterial benötigt wird.

In einer weiteren bevorzugten Ausgestaltung sind die freien Endabschnitte in axialer Verlängerung des Stents miteinander verdrillt.

Diese Maßnahme hat den Vorteil, daß die freien Endabschnitte weder radial nach außen abgebogen sind, so daß sie sich beim Einführen mit der Gefäßinnenwand nicht verhaken können und das Einführen des Stents erleichtern, noch sind sie nach innen abgebogen, so daß sie den Durchfluß von Blut nicht behindern.

In einer weiteren bevorzugten Ausgestaltung sind jeweils diejenigen Endabschnitte miteinander verdrillt, die von demselben Kreuzungspunkt des Geflechts am ersten Ende des Stents überstehen.

Durch diese Maßnahme können die freien Endabschnitte einerseits in axialer Verlängerung des Stents miteinander verdrillt werden, andererseits fixieren die verdrillten Endabschnitte die äußersten Verkreuzungspunkte des Geflechts und schließen das Geflecht an dem ersten Ende.

Bei einer bevorzugten Ausgestaltung des erfindungsgemäßen Stents weist der Körper an dem Ende, an dem die fadenförmigen Elemente umgelenkt sind, eine sich trompetenartig erweiternde Krone auf.

Beim Implantieren des Stents in dem Blutgefäß bewirkt die sich trompetenartig erweiternde Krone eine zusätzliche, besonders wirksame Verankerung des erfindungsgemäßen Stents in dem Blutgefäß. Wird der erfindungsgemäße Stent derart in das Blutgefäß implantiert, daß die sich trompetenartig erweiternde Krone das stromaufwärts gerichtete Ende des erfindungsgemäßen Stents in dem Gefäß darstellt, drückt das in dem Gefäß strömende Blut die umgelenkten und trompetenartig radial nach außen stehenden Abschnitte fest gegen die Wand des Blutgefäßes, wodurch der erfindungsgemäße Stent an dieser Stelle fest mit der Gefäßwand verankert ist und sich nicht verschieben kann. Es ist daher nicht mehr wie bei den herkömmlichen Stents erforderlich, die überstehenden freien Endabschnitte radial nach außen zu biegen, um den Stent mit diesen freien Endabschnitten in dem Gefäß zu verankern. Dadurch wird bei dem erfindungsgemäßen Stent auf vorteilhafte Weise eine Verletzung der Gefäßinnenwand durch die freien Endabschnitte vermieden. Der erfindungsgemäße Stent kann somit auf vorteilhafte Weise atraumatisch in dem Gefäß verankert werden. Die Krone kann dadurch bewerkstelligt werden, daß die hochstehenden Umlenkenden der Fäden nach außen gebogen werden.

In einer weiteren bevorzugten Ausgestaltung sind die fadenförmigen Elemente zwischen dem ersten Ende und dem anderen Ende schraubenlinienförmig geführt.

Durch diese Maßnahme wird jedes fadenförmige Element über dem gesamten Umfang des Stents mit jedem anderen fadenförmigen Element verflochten, so daß ein vorteilhaft in sich fixiertes, jedoch elastisches Geflecht gebildet wird.

Dabei ist es bevorzugt, wenn jedes fadenförmige Element von dem ersten Ende des Stents ausgehend in einer ersten Schraubenlinie zu dem anderen Ende geführt ist, und nach der Umlenkung in einer die erste Schraubenlinie kreuzenden zweiten Schraubenlinie zu dem ersten Ende zurückgeführt ist.

Durch diese Maßnahme wird das Geflecht aus zwei miteinander verflochtenen sich kreuzenden Systemen von fadenförmigen Elementen gebildet, wobei der Vorteil erzielt wird, daß beide Systeme an den Umlenkungsstellen fest miteinander verbunden sind. Dadurch entsteht auf vorteilhafte Weise ein an einem Ende des Stents geschlossenes Geflecht mit einer Vielzahl polygonförmiger Zellen, die ihre polygonförmige Struktur auch unter Belastung dauerhaft beibehalten.

In einer weiteren bevorzugten Ausgestaltung ist der Körper aus sechs fadenförmigen Elementen aufgebaut.

Bei dieser Anzahl fadenförmiger Elemente nehmen somit deren zwölf Enden, die von dem Umlenkungspunkten wegführen, an der Bildung des Geflechts teil. Bei den herkömmlichen Stents werden zur Herstellung desselben Geflechts dagegen zwölf einzelne fadenförmige Elemente benötigt, die miteinander verflochten werden müssen.

In einer weiteren bevorzugten Ausgestaltung bestehen die fadenförmigen Elemente aus Nitinol-Draht.

Nitinol ist eine Nickel-Titan-Legierung, die sich aufgrund ihres Formgedächtnisses als Material für Stents besonders eignet.

Weitere Vorteile ergeben sich aus der nachfolgenden Beschreibung der beigefügten Zeichnung.

Ein Ausführungsbeispiel der Erfindung wird hiernach mit Bezug auf die beigefügte Zeichnung näher beschrieben. Es zeigen:
- Fig. 1: eine Seitenansicht eines Stents;
- Fig. 2: den Stent in Fig. 1 in einer abgewickelten Darstellung, nach dem dieser entlang der Mantellinie II-II in Fig. 1 aufgeschnitten worden ist;
- Fig. 3: einen vergrößerten Ausschnitt eines fadenförmigen Elementes des Stents in Fig. 1 im Bereich einer in dem fadenförmigen Element gebildeten Schlaufe;
- Fig. 4: einen vergrößerten Ausschnitt aus dem Geflecht des Stents in Fig. 1;
- Fig. 5: einen Wickelkörper zur Verwendung bei der Herstellung des Stents in Fig. 1; und
- Fig. 6a) bis d): Prinzipdarstellungen verschiedener Arbeitsschritte eines Verfahrens zur Herstellung des Stents in Fig. 1.

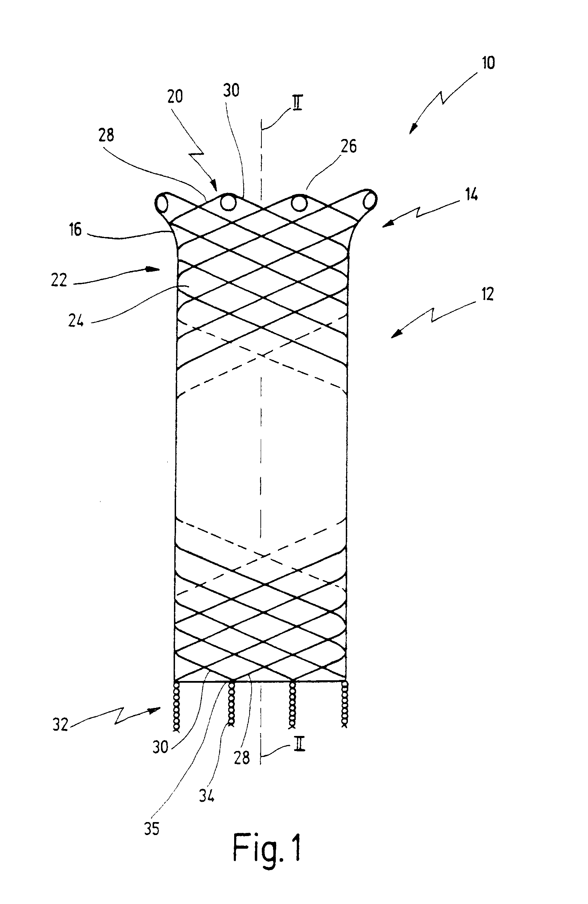

In Fig. 1 ist ein mit dem allgemeinen Bezugszeichen 10 versehener Stent dargestellt. Der Stent 10 wird in ein Blutgefäß wie eine Vene oder Arterie im menschlichen Körper mittels eines hier nicht dargestellten Applikators implantiert.

Der Stent 10 weist einen hohlzylindrischen Körper 12 auf, der somit in Längsrichtung beidseitig für den Durchgang von Blut offen ist.

An seinem ersten, in Fig. 1 oberen Ende 14 weist der Körper 12 eine sich trompetenartig erweiternde Krone 16 auf.

Der Körper 12 wird aus mehreren umfänglich gegeneinander versetzten fadenförmigen Elementen 20 gebildet. Der in Fig. 1 dargestellte Stent 10 besteht insgesamt aus sechs fadenförmigen Elementen 20, wobei jedoch die Anzahl an fadenförmigen Elementen 20 nicht kritisch ist. Die fadenförmigen Elemente 20 bestehen aus Nitinol-Draht.

Die fadenförmigen Elemente 20 sind miteinander zu einem Geflecht 22 verflochten, das eine Vielzahl polygonförmiger Zellen 24, im vorliegenden Fall rhombenförmige Zellen aufweist. Die Ausbildung des Geflechts 22 wird noch genauer an späterer Stelle beschrieben.

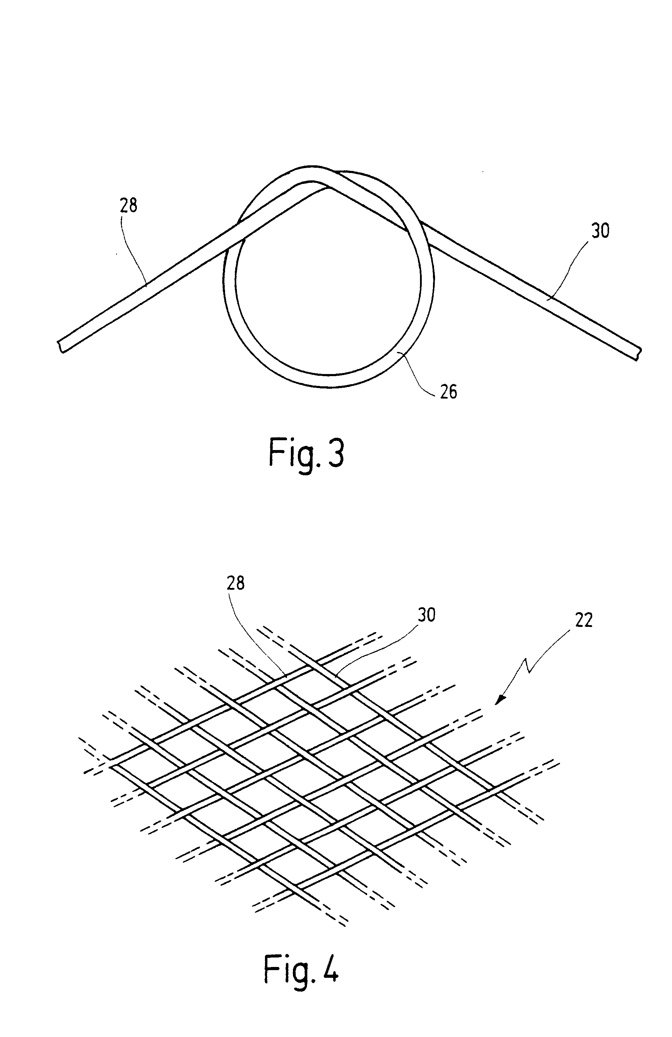

Im Bereich des ersten Endes 14, d.h. an der Krone 16 ist jedes fadenförmige Element 20 durch eine Schlaufe 26 umgelenkt, die in Fig. 3 in stark vergrößertem Maßstab dargestellt ist.

Von jeder Schlaufe 26 führen ein erstes Ende 28 und ein zweites Ende 30 des jeweils selben fadenförmigen Elementes 20 weg. Die Schlaufe 26 ist dabei so ausgebildet, daß die Enden 28 und 30 miteinander eine Schenkelfeder bilden, demnach wie bei einer Wäscheklammer.

Die Schlaufe 26 ist hier dadurch gebildet, daß die Enden 28 und 30 desselben fadenförmigen Elementes 20 einmal miteinander zu einem Knoten verschlungen sind (vgl. Fig. 3). Der Durchmesser der Schlaufen 26 ist nicht kritisch, d.h. der Knoten kann mehr oder weniger weit zugezogen sein. Anstelle des Knotens kann auch eine einfache geringfügig gespreizte U-förmige Umlenkung der fadenförmigen Elemente 20 die Schlaufe 26 bilden.

An einem zweiten, in Fig. 1 unteren Ende 32 des Körpers 12 sind überstehende freie Endabschnitte 34 der Enden 28 und 30 der fadenförmigen Elemente 20 unauflösbar miteinander verbunden, in dem gezeigten Ausführungsbeispiel sind diese dazu miteinander verdrillt, und zwar paarweise, und somit fixiert. Es sind diejenigen freien Endabschnitte 34 jeweils miteinander verdrillt, die von demselben Kreuzungspunkt 35 des Geflechts 22 überstehen. Die verdrillten Endabschnitte 34 stehen in axialer Verlängerung des Stents 10 von diesem ab. Entsprechend der Anzahl von sechs fadenförmigen Elementen 20 und damit sechs Enden 28 und sechs Enden 30 weist der Stent 10 sechs verdrillte überstehende Endabschnitte 34 auf.

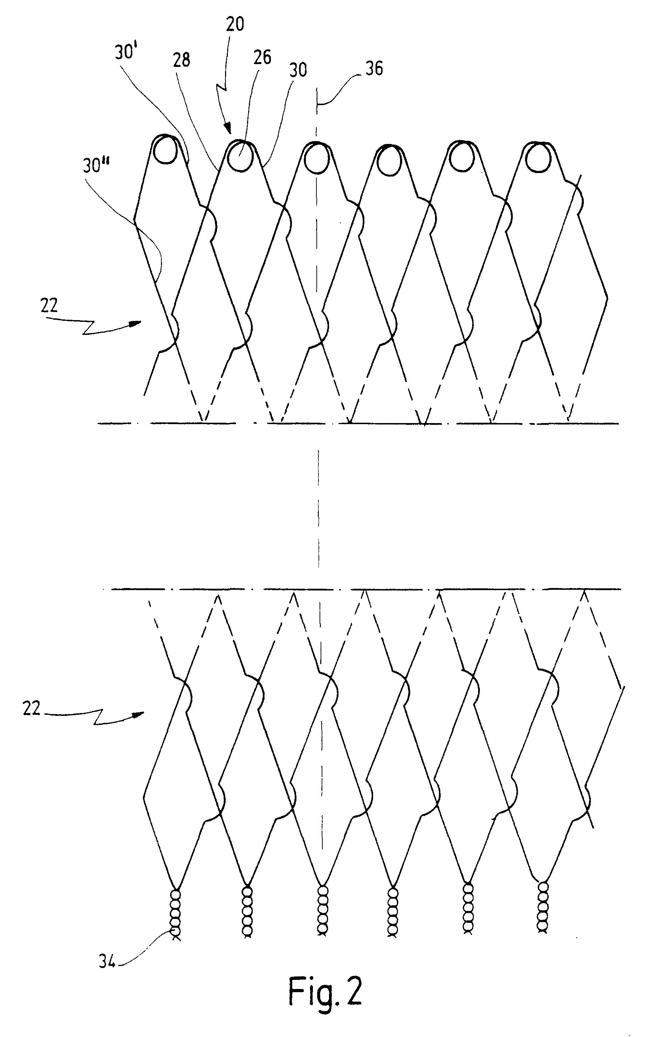

Während in Fig. 1 in der Seitenansicht nur vier Schlaufen 26 und vier überstehende Endabschnitte 34 zu sehen sind, sind in der in Fig. 2 gezeigten abgewickelten Darstellung alle sechs Schlaufen 26 der sechs fadenförmigen Elemente 20 sowie alle sechs verdrillten überstehenden Endabschnitte 34 der fadenförmigen Elemente 20 zu sehen.

In Fig. 2 erkennt man zunächst, daß die Schlaufen 26 so angeordnet sind, daß die Enden 28 und 30 der fadenförmigen Elemente 20 vom oberen Umfang der Schlaufe 26 wegführen, d.h., daß sich der Knoten am oberen Umfangsbereich der Schlaufe 26 befindet.

Das Geflecht 22 ist so ausgebildet, daß jedes fadenförmige Element 20 von dem Ende 32 des Stents 10 ausgehend in einer ersten Schraubenlinie zu dem anderen Ende 14 geführt ist und dort zu der Schlaufe 26 gelegt ist. Das fadenförmige Element 20 ist über die Schlaufe 26 in Richtung des Endes 32 umgelenkt und in einer die erste Schraubenlinie kreuzenden zweiten Schraubenlinie zu dem ersten Ende 32 zurückgeführt.

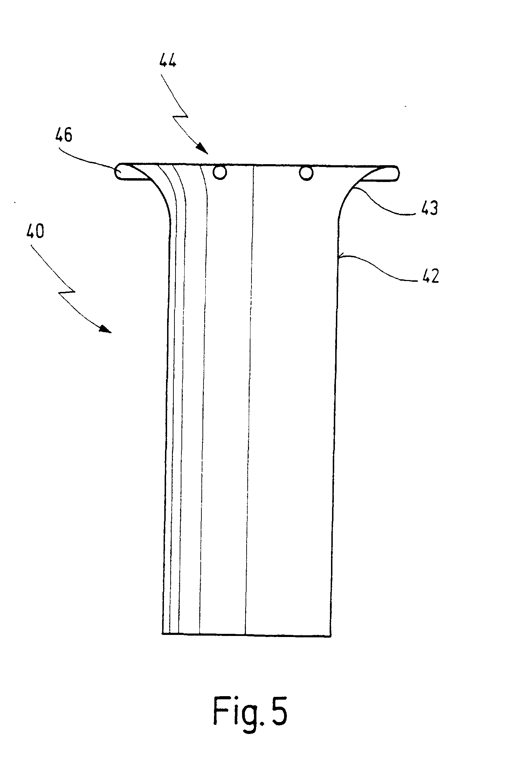

Dies bedeutet, daß die Enden 28 in einer zu einer Mantellinie 36 des Körpers 12 ersten schrägen Richtung von der jeweiligen Schlaufe 26 wegführen, während die Enden 30 in einer bezüglich der Mantellinie 36 dazu spiegelbildlichen Richtung von der Schlaufe 26 wegführen. Jedes Ende 28 jedes fadenförmigen Elementes 20 ist mit allen Enden 30 aller fadenförmigen Elemente 20 zur Bildung des Geflechts 22 verkreuzt. Dazu ist beispielhaft in Fig. 2 dargestellt, daß das Ende 28 von der Schlaufe 26 aus abwechselnd unter einem Ende 30' durch und anschließend über ein nächstes Ende 30' ' verläuft, usw. Die Enden 28 und 30 der fadenförmigen Elemente 20 bilden zwischen dem oberen Ende 14 und dem unteren Ende 32 des Körpers 12 somit zwei miteinander verkreuzte, gegensinnig schraubenlinienförmige Fadenlagen. Das in Fig. 2 und 4 dargestellte Geflecht 22 ist somit ein Geflecht mit einer Leinwand-Bindung.

Die verdrillten freien Endabschnitte 34 sind nicht zwingend Endabschnitte desselben fadenförmigen Elements 20, sondern können zu verschiedenen fadenförmigen Elementen 20 gehören.

In Fig. 5 ist ein Wickelkörper 40 dargestellt, mit dem der Stent 10 hergestellt wird. Der Wickelkörper 40 weist eine Außenkontur 42 auf, die der Kontur des Stents 10 entspricht. Dies bedeutet, daß der Wickelkörper 40 ebenso wie der Stent 10 einen sich radial erweiternden Abschnitt 43 aufweist. Die Länge des Wickelkörpers 40 entspricht der Länge des Stents 10.

Im Bereich des Abschnitts 43 weist der Wickelkörper 40 Haltemittel 44 zum Befestigen der Schlaufen 26 auf. Die Haltemittel 44 sind als umfänglich um den Wickelkörper 40 verteilt angeordnete und von diesem vorstehende Stifte 46 ausgebildet, von denen entsprechend der Anzahl der fadenförmigen Elemente 20 sechs vorhanden sind.

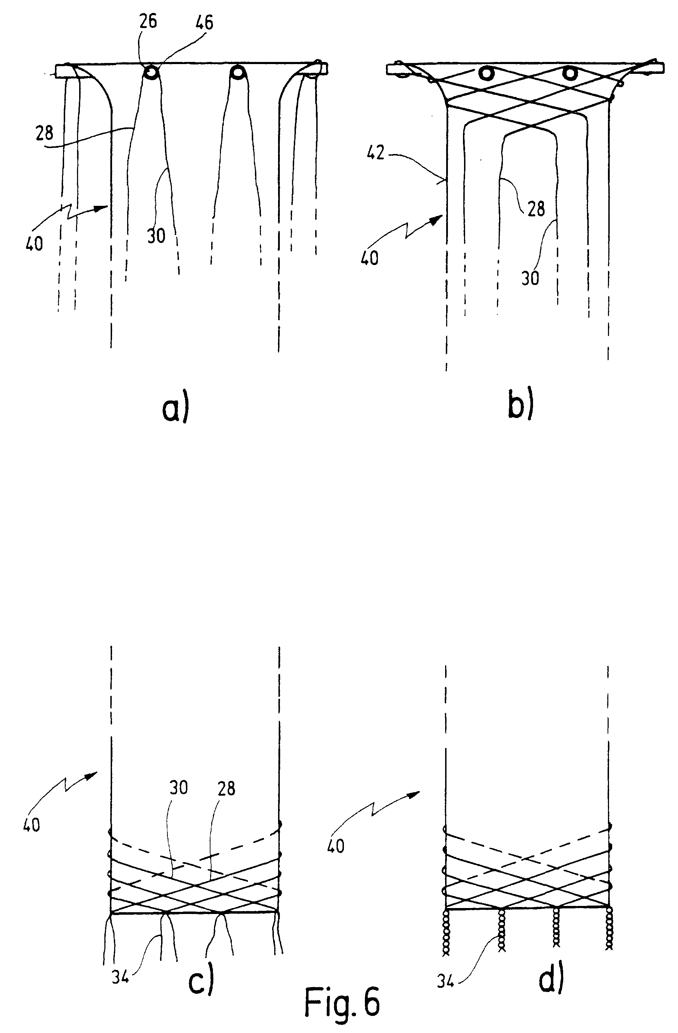

Mit Bezug auf Fig. 6 wird nun ein Verfahren zum Herstellen des Stents 10 beschrieben.

Zunächst wird an jedem fadenförmigen Element 20 eine Schlaufe 26, wie sie in Fig. 3 dargestellt ist, gebildet, und zwar etwa mittig bezüglich der Länge der einzelnen fadenförmigen Elemente 20. Die fadenförmigen Elemente 20 können bereits in Abhängigkeit von der Länge, dem Durchmesser des Körpers 12 und der Dichte des Geflechts 22 des herzustellenden Stents 10 zugeschnitten sein, jedoch empfiehlt es sich, die fadenförmigen Elemente 20 mit einer entsprechenden geringen Überlänge zuzuschneiden.

Die fadenförmigen Elemente 20 werden an den Stiften 46 des Wikkelkörpers 40 festgelegt, indem die Schlaufen 26 auf die Stifte 46 aufgeschoben werden. Die Enden 28 und 30 der fadenförmigen Elemente 20 hängen dann von den Stiften 46 herab, wie in Fig. 6a) dargestellt ist.

Im nächsten Schritt werden die Enden 28 und 30 gemäß der in Fig. 2 und 4 dargestellten Weise zur Bildung des Geflechts 22 miteinander verflochten.

Dies wird fortgesetzt, bis das Geflecht 22 die für den Stent 10 vorbestimmte Länge erreicht hat (vgl. Fig. 6c)). Die über die Länge des Wickelkörpers 40 hinaus überstehenden freien Endabschnitte 34 der fadenförmigen Elemente 20 werden anschließend paarweise miteinander verdrillt. Die überstehenden Endabschnitte 34 können auf gleiche Länge abgeschnitten werden, bevor sie miteinander verdrillt werden, oder nachdem sie miteinander verdrillt worden sind.

Der so hergestellte Stent 10 wird danach von dem Wickelkörper 40 nach oben abgezogen. Damit die Stifte 46 dazu mit den Schlaufen 26 außer Eingriff kommen, kann vorgesehen sein, daß sich die Stifte 46 nach innen in den Wickelkörper 40 drücken lassen.

Der so hergestellte Stent 10 kann dann zusätzlich mit einer Hülle, bspw. aus Latex, überzogen werden und ist dann zur Implantation in ein Blutgefäß bereit.

Der Stent 10 wird so in das Gefäß implantiert, daß die Krone 16 das stromaufwärtige Ende des Stents 10 in dem Gefäß bildet, d.h. daß das Blut in die Krone 16 einströmt und aus dem Ende 32 ausströmt. Die Strömung des Blutes bewirkt, daß die Schlaufen 26 an die Gefäßwand gedrückt werden, ohne in diese einzudringen und die Gefäßwand zu verletzen, wodurch der Stent 10 sicher in dem Gefäß verankert ist.

## Patentansprüche

1. Stent zur Implantation im menschlichen Körper, insbesondere in Blutgefäße, mit einem hohlzylindrischen Körper (12), der aus mehreren umfänglich gegeneinander versetzten fadenförmigen Elementen (20) aufgebaut ist, die zu einem Geflecht (22) mit einer Vielzahl polygonförmiger Zellen (24) verflochten sind, wobei jedes fadenförmige Element (20) von einem ersten Ende (32) des Stents (10) ausgehend entsprechend dem Geflecht (22) zum anderen Ende (14) des Stents (10) geführt ist, dort in Richtung erstes Ende (32) umgelenkt und wieder zum ersten Ende entsprechend dem Geflecht (22) rückgeführt ist, wobei freie Endabschnitte (34) der fadenförmigen Elemente (20) an dem ersten Ende (32) auflösungssicher miteinander verbunden sind, und wobei jedes fadenförmige Element (20) an dem Ende (14), an dem es umgelenkt ist, zu einer Schlaufe (26) gelegt ist, **dadurch gekennzeichnet, daß** die Schlaufe (26) so ausgebildet ist, daß die beiden von jeder Schlaufe (26) wegführenden Enden (28, 30) des zugehörigen fadenförmigen Elementes (20) im Bereich der Schlaufe (26) eine Art Schenkelfeder bilden.

2. Stent nach Anspruch 1, **dadurch gekennzeichnet, daß** die fadenförmigen Elemente (20) in Form einer Leinwand-Bindung zu dem Geflecht (22) verflochten sind.

3. Stent nach Anspruch 1 oder 2, **dadurch gekennzeichnet, daß** die Schlaufe (26) als Knoten ausgebildet ist.

4. Stent nach einem der Ansprüche 1 bis 3, **dadurch gekennzeichnet, daß** die freien Endabschnitte (34) miteinander verdrillt sind.

5. Stent nach Anspruch 4, **dadurch gekennzeichnet, daß** die freien Endabschnitte (34) in axialer Verlängerung des Stents (10) miteinander verdrillt sind.

6. Stent nach Anspruch 4 oder 5, **dadurch gekennzeichnet, daß** jeweils diejenigen Endabschnitte (34) miteinander verdrillt sind, die von einem selben Kreuzungspunkt des Geflechts (22) am ersten Ende (32) des Stents (10) überstehen.

7. Stent nach einem der Ansprüche 1 bis 6, **dadurch gekennzeichnet, daß** der Körper (12) an dem Ende (14), an dem die fadenförmigen Elemente (20) umgelenkt sind, eine sich trompetenartig erweiternde Krone (16) aufweist.

8. Stent nach einem der Ansprüche 1 bis 7, **dadurch gekennzeichnet, daß** die fadenförmigen Elemente (20) zwischen dem ersten Ende (32) und dem anderen Ende (14) schraubenlinienförmig geführt sind.

9. Stent nach Anspruch 8, **dadurch gekennzeichnet, daß** jedes fadenförmige Element (20) von den erstem Ende (32) des Stents (10) ausgehend in einer ersten Schraubenlinie zu dem anderen Ende (14) geführt ist, und nach der Umlenkung in einer die erste Schraubenlinie kreuzenden zweiten Schraubenlinie zu dem ersten Ende (32) zurückgeführt ist.

10. Stent nach einem der Ansprüche 1 bis 9, **dadurch gekennzeichnet, daß** der Körper (12) aus sechs fadenförmigen Elementen (20) aufgebaut ist.

11. Stent nach einem der Ansprüche 1 bis 10, **dadurch gekennzeichnet, daß** die fadenförmigen Elemente (20) aus Nitinol-Draht bestehen.

## Claims

1. A stent for implantation in a human body, in particular in blood vessels, comprising a hollow-cylindrical body (12) composed of a plurality of circumferentially offset thread-like elements (20) that are interwoven to form a weave (22) with a plurality of polygon-shaped cells (24), wherein each thread-like element (20) is guided from a first end (32) of the stent (10), according to the weave (22), to the other end (14) of the stent (10), where it is turned back toward the first end (32) and returned to the latter according to the weave (22), wherein free end portions (34) of the thread-like elements (20) are connected one with the other at the first end (32) in disengageable-proof fashion, and wherein each thread-like element (20), at the end (14), where it is turned back, is formed to a loop (26), **characterized in that** the loop (26) is configured such that the two ends of the associated thread-like element (20), that lead away from the loop (26), form sort of a leg spring in the area of the loop (26).

2. The stent of Claim 1, **characterized in that** the thread-like elements (20) are interwoven to the weave (22) in the form of a plain weave.

3. The stent of Claim 1 or 2, **characterized in that** the loop (26) is configured as a knot.

4. The stent of anyone of Claims 1 through 3, **characterized in that** the free end portions (34) are connected with one another by twisting.

5. The stent of Claim 4, **characterized in that** the free end portions (34) are twisted to form an axial extension of the stent (10).

6. The stent of Claim 4 or 5, **characterized in that** those end portions (34) are twisted with one another which project from a same crossing point of the weave (22) at the first end (32) of the stent (10).

7. The stent of anyone of Claims 1 through 6, **characterized in that** the body (12) exhibits a crown (16) widening in trumpet shape at that end (14) where the thread-like elements (20) are turned back.

8. The stent of anyone of Claims 1 through 7, **characterized in that** the thread-like elements (20) are guided between the first end (32) and the other end (14) along a helical line.

9. The stent of Claim 8, **characterized in that** each thread-like element (20) is guided from the first end (32) of the stent (10) along a first helical line to the other end (14) ,where it is turned back and returned along a second helical line, crossing the first helical line, back to the first end (32).

10. The stent of anyone of Claims 1 through 9, **characterized in that** the body (12) is made up from six thread-like elements (20).

11. The stent of anyone of Claims 1 through 10, wherein the thread-like elements (20) consist of nitinol wire.

## Revendications

1. Stent pour implantation dans le corps humain, en particulier dans des vaisseaux sanguins, avec un corps cylindrique creux (12), qui est construit avec plusieurs éléments en forme de fils (20) placés en quinconce les uns contre les autres en circonférence, qui sont entrelacés avec plusieurs cellules (24) en forme de polygone pour former un treillis (22), chaque élément en forme de fil (20) étant amené d'une première extrémité (32) du stent (10) en suivant le treillis (22) jusqu'à l'autre extrémité (14) du stent, où il est retourné en direction de la première extrémité (32) et ramené de nouveau à la première extrémité en suivant le treillis (22), les extrémités libres (34) des éléments en forme de fil (20) étant nouées ensemble au niveau de la première extrémité (32) de manière indissoluble, et chaque élément en forme de fil (20) à l'extrémité (14) au niveau de laquelle il est retourné étant formé en une boucle (26), **caractérisée en ce que** la boucle (26) est formée de telle sorte que les deux extrémités (28, 30) s'écartant de chaque boucle (26) de l'élément en forme de fil (20) concerné forment dans la zone de la boucle (26) une sorte de ressort de flexion.

2. Stent selon la revendication 1, **caractérisé en ce que** les éléments en forme de fil (20) sont entrelacés en forme d'armure de toile pour former le treillis (22).

3. Stent selon la revendication 1 ou 2, **caractérisé en ce que** la boucle (26) est formée comme un noeud.

4. Stent selon l'une des revendications 1 à 3, **caractérisée en ce que** les extrémités libres (34) sont torsadées ensemble.

5. Stent selon la revendication 4, **caractérisé en ce que** les extrémités libres (34) sont torsadées ensemble en une prolongation axiale du stent (10).

6. Stent selon la revendication 4 ou 5, **caractérisé en ce que** les extrémités libres (34) qui dépassent d'un même point de croisement du treillis (22) à la première extrémité (32) du stent (10) sont à chaque fois torsadées ensemble.

7. Stent selon l'une des revendications 1 à 6, **caractérisé en ce que** le corps (12), à l'extrémité (14) à laquelle les éléments en forme de fil (20) sont retournés, présente une couronne (16) s'élargissant en trompette.

8. Stent selon l'une des revendications 1 à 7, **caractérisé en ce que** les éléments en forme de fil (20) sont amenés en forme d'hélice entre la première extrémité (32) l'autre extrémité (14).

9. Stent selon la revendication 8, **caractérisé en ce que** chaque élément en forme de fil (20) est amené en une première hélice depuis la première extrémité (32) du stent (10) jusqu'à l'autre extrémité (14) et, après le retournement, est ramené en une seconde hélice croisant la première hélice jusqu'à la première extrémité (32).

10. Stent selon l'une des revendications 1 à 9, **caractérisé en ce que** le corps (12) est constitué de six éléments en forme de fil (20).

11. Stent selon l'une des revendications 1 à 10, **caractérisé en ce que** les éléments en forme de fil (20) sont constitués de fil métallique en nitinol.
